# EUROPÄISCHE PATENTANMELDUNG

(11) **EP 2 460 481 A1**
(43) Veröffentlichungstag der Anmeldung: **06.06.2012**
(21) Anmeldenummer: 10193333.1
(22) Anmeldetag: 01.12.2010
(51) Int. Cl.: A61B 17/70

(54) **Fusionsimplantat für Facettengelenke**

(71) Anmelder: FACET-LINK Inc., Rockaway NJ 07866 (US)
(72) Erfinder: Jensen, Harm-Iven, 24214 Noer (DE); Link, Helmut D., 22397 Hamburg (DE)
(74) Vertreter: Glawe, Delfs, Moll

(57) **Zusammenfassung**

Fusionsimplantat für ein Facettengelenk, umfassend ein Tragemodul (1) und ein Fusionsmodul (6), wobei das Fusionsmodul (6) an dem Tragmodul (1) angeordnet ist und einen Halter für transfacettale Befestigungsmittel (65) aufweist, wobei das Tragmodul (1) ein Expansionselement (2,3) und Backenelemente (4,4') umfasst, die an voneinander wegweisenden Außenseiten Anlageflächen (43) für die Lamina (93) aufweisen und die längsverschieblich an einer Führung so angeordnet sind, dass ein Abstand der Fusionsmodule (6) mittels des Expansionselements veränderlich ist. Das Tragmodul bildet eine sichere und präzise positionierbare Verankerung für das Fusionsmodul. Weiter bewirkt das Tragmodul eine Augmentation, so dass eine Therapie auch bei durch Defekte geschwächten Wirbelkörpern ermöglicht ist.

## Beschreibung

Die Erfindung betrifft ein Fusionsimplantat für Facettengelenke, das ein Fusionsmodul zur Verbindung der Facettengelenke benachbarter Wirbel und ein das Fusionsmodul halterndes Tragmodul aufweisen.

Die Wirbelsäule bildet ein zentrales Strukturelement des menschlichen Skeletts. Sie umfasst eine Vielzahl von Wirbeln, die für die Übertragung von Lasten übereinander angeordnet und zur Ermöglichung von Bewegungen gelenkig miteinander verbunden sind. Die Wirbel der Wirbelsäule sind nicht identisch, sondern unterscheiden sich in ihrer Gestalt je nach ihrer Anordnung an der Wirbelsäule. Sie weisen jedoch einige Gemeinsamkeiten auf. So weist jeder Wirbel einen massiven Wirbelkörper mit zwei seitlich nach hinten vorstehenden knöchernen Vorsprüngen (Pedikel) auf, die wiederum in ihrem hinteren Teil über einen knöchernen Bogen verbunden sind. Im Verbindungsbereich ist dieser knöcherne Bogen als breite Platte (Lamina) ausgebildet, und weist in seiner Mitte einen nach hinten abragenden spinalen Vorsprung auf. Der spinale Vorsprung (Prozessus spinalis) wie auch zwei weitere transverse Vorsprünge an den Seitenflächen der Pedikel bilden Anlenkpunkte für Muskeln und Bänder. In dem Bereich, in dem die Pedikel in die breite Lamina übergehen, sind auf jeder Seite des Wirbels ein oberer und ein unterer Gelenkvorsprung angeordnet. Sie bilden jeweils Teil eines Facettengelenks mit einem benachbarten unteren beziehungsweise oberen Wirbel. Weiter ist zur Lastübertragung der Wirbel vorgesehen, dass zwischen den Wirbelkörpern benachbarter Wirbel jeweils eine Bandscheibe angeordnet ist, die den Zwischenraum zwischen den verhältnismäßig ebenen Deckflächen benachbarter Wirbelkörper ausfüllt. Der von den Rückseiten des Wirbelkörpers und dem knöchernen Bogen (Wirbelbogen) umgrenzter Bereich bildet einen Hohlraum, in welchem parallel zur Wirbelsäule laufende Nervenstränge aufgenommen sind.

Aufgrund von Degeneration der Wirbelsäule kommt es häufig zu Rückenbeschwerden. Hierbei liegt eine der Hauptursachen für Rückenschmerzen in der Interaktion zwischen zwei benachbarten Wirbeln. Dies betrifft insbesondere Bandscheiben als eine Hauptursache, aber in einem beträchtlichen Anteil der Fälle umfasst die Pathologie zumindest auch Facettengelenke. Aufgrund von Verschleiß oder Krankheit kann die für die Facettengelenke bewirkte gelenkige Verbindung zweier benachbarter Wirbel beschädigt sein. Dies kann zu Bewegungseinschränkungen, Schmerzen bis hin zu einem Verlust der Mobilität führen. Es sind verschiedene Ansätze zur Therapie bekannt geworden. Insbesondere kann eine deutliche Verbesserung erreicht werden, indem das Facettengelenk stabilisiert wird. In vielen Feldern folgt dies in der Weise, dass das Facettengelenk durch eine feste Verbindung immobilisiert wird. Hierbei spricht man von einer Fusion des Facettengelenks.

Aus der WO 2009/094629 A1 ist ein Fusionsimplantat bekannt geworden, das lange Knochenschrauben umfasst, welche durch die beiden ein Facettengelenk bildenden Facetten durchgeschraubt wird. Die Schraube ist als Kompressionsschraube ausgeführt. Sie zieht die miteinander zusammenwirkenden Hälften des Facettengelenks zusammen, so dass das Gelenk immobilisiert ist. Um die dafür erforderlichen Kräfte auf den Wirbelkörper übertragen zu können, ist der Schraubenkopf mit einer schwenkbeweglich angeordneten gesonderten Auflagehülse versehen. Mit ihrer Schwenkbeweglichkeit kann die transfacettale Kompressionsschraube verschiedene Winkelstellungen zu der durch die Auflagehülse bestimmten Auflageebene einnehmen. Man spricht von einer polyaxialen Anordnung der transfacettalen Schraube. Für jedes der beiden Facettengelenke eines Wirbelkörpers (auf einer linken beziehungsweise rechten Seite) ist eine eigene Schraube vorgesehen. Das bekannte Fusionsimplantat bietet den Vorteil verhältnismäßig einfacher Implantierbarkeit, da es nur geringe Abmessungen aufweist und daher auch mittels minimal invasiver Chirurgie implantiert werden kann. Allerdings erfordert dieses bekannte Fusionsimplantat eine verhältnismäßig starke und intakte Knochenstruktur am Wirbelkörper, insbesondere im Bereich der Auflagefläche der schwenkbeweglichen Krause um den Schraubenkopf.

Aus der US 2005/0192572 A ist ein Fusionsimplantat bekannt, das ebenfalls polyaxial geführte transfacettale Facettenschrauben zur Fusion der beiden Gelenkhälften eines Facettengelenks vorsieht. Im Unterschied zu der vorstehend beschriebenen Implantationsanordnung weist diese Ausführungsform zusätzlich eine Quertraverse auf, an der die beiden transfacettalen Fusionsschrauben (für das linke und rechte Facettengelenk eines Wirbelkörpers) über Gleitverbinder geführt sind. Die Gleitverbinder sind so ausgestaltet, dass sie in ihrer Position fixiert sind durch Klemmkräfte, die beim Einbringen und Anziehen der Facettenschraube entstehen. Der Vorteil dieses Implantationsanordnung liegt darin, dass eine stabile Positionierung der einen Facettenschraube relativ zu der auf der anderen Seite des Wirbelkörpers erreicht wird. Allerdings besteht keine Kontrolle über die absolute Lage der Fusionsschrauben in Bezug auf den Wirbelkörper. Außerdem erfordert auch diese Implantationsanordnung eine verhältnismäßig starke und intakte Knochenstruktur des Wirbelkörpers.

Der Erfindung liegt die Aufgabe zugrunde, ein verbessertes Fusionsimplantat der eingangsgenannten Art zu schaffen, das die oben genannten Nachteile vermeidet.

Die erfindungsgemäße Lösung liegt in den Merkmalen der unabhängigen Ansprüche. Vorteilhafte Weiterbildungen sind Gegenstand der abhängigen Ansprüche.

Bei einem Fusionsimplantat für ein Facettengelenk, umfassend ein Tragmodul und ein Fusionsmodul, wobei das Fusionsmodul an dem Tragmodul angeordnet ist und einen Halter für transfacettale Befestigungsmittel aufweist, ist erfindungsgemäß vorgesehen, dass das Tragmodul ein Expansionselement und Backenelemente umfasst, die an voneinander wegweisenden Außenseiten Anlageflächen für die Lamina aufweisen und die längsverschieblich an einer Führung so angeordnet sind, dass ein Abstand der Fusionsmodule mittels des Expansionselements veränderlich ist.

Die Erfindung beruht auf dem Gedanken, ein Fusionsimplantat zu schaffen, bei dem die Funktion des Haltens und der Positionierung an dem Wirbelkörper entkoppelt ist von der Funktion des eigentlichen Einwirkens auf die Facettengelenke. Mit dem Tragmodul wird eine sichere und präzise positionierbare Verankerung des Fusionsimplantats an einem Wirbelkörper, insbesondere an Laminaschnittflächen, erreicht. Es wird eine solide und feste Plattform geschaffen, so dass die die Fusion bewirkenden Komponenten sowohl absolut bezogen auf den Wirbelkörper wie auch relativ zueinander präzise und sicher positioniert werden können. Weiter bietet die Modulbauweise den Vorteil, dass sie sich insbesondere gut zur Implantation mittels minimal invasiver Chirurgie eignet.

Außerdem erreicht man mit dem Tragmodul eine Augmentation, so dass das erfindungsgemäße Fusionsimplantat sich insbesondere zur Anwendung an Wirbelkörper mit auch größeren Defekten, insbesondere im Bereich der Lamina, eignet. Die Erfindung bietet damit den großen Vorteil, auch dann angewendet werden zu können, wenn der Wirbelkörper durch vorhergehende Dekompressions-Therapie geschwächt ist. Gerade bei dieser in der Praxis sehr häufig vorkommenden Therapie war eine Facettengelenksfusion bisher kontra-indiziert. Die Erfindung ermöglicht dies nun und erhöht somit beträchtlich die Zahl der Patienten, denen geholfen werden kann.

Die Erfindung verknüpft damit auf besondere Weise Vorteile hinsichtlich breiter Verwendbarkeit dank Augmentation auch bei Wirbelkörpern mit beträchtlichen Defekten und einer präziseren Positionierung am Wirbelkörper mit Patientenschonung dank minimal invasiver Implantation. Die Facettenfusion als "Goldstandard" kann so mit anderen Therapietechniken kombiniert werden.

Zuerst seien einige verwendete Begriffe erläutert:
Unter "transfacettal" wird ein Durchquerung des Facettengelenks verstanden. Das Facettengelenk umfasst hierbei eine untere Facette des Wirbels, an dem das erfindungsgemäße Implantat anzuordnen ist, und eine obere Facette des darunter liegenden Wirbels; sie bilden gemeinsam das Facettengelenk.
Unter "cephalad" wird eine Richtung verstanden, die bezogen auf die implantierte Position in Richtung nach oben, also zum Kopf des Patienten weist. Dementsprechend wird unter caudal die entgegengesetzte Richtung verstanden, die also vom Kopf weg nach unten weist.

Das Fusionsmodul weist vorzugsweise ein Hauptlager an einer oberen Facette und ein Gegenlager an einer unteren Facette des zu fusionierenden Facettengelenks auf. Die Einleitung der Fusionskräfte kann damit auf beiden Seiten in definierter und letztlich Knochensubstanz schonender Weise erfolgen. Das Gegenlager kann als gesondertes Element an der jenseitigen Facette angeordnet sein, oder es kann einstückig mit dem die eigentliche Fusion bewirkenden Element ausgebildet sein, beispielsweise als Knochengewinde einer transfacettalen Schraube als Befestigungselement. Die transfacettale Schraube kann kanüliert oder solide ausgeführt sein.

Das Hauptlager ist vorzugsweise schwenkbar. Damit wird kann die Richtung, in der die Facettenfusion erfolgt, mithin also die funktionale Achse verändert werden. Eine solche polyaxiale Lagerung ermöglicht eine verbesserte Anpassung an die individuellen anatomischen Verhältnisse. Bewährt hat sich ein Verstellbereich von +/- 15 Grad in alle Richtungen. Bei einer zweckmäßigen Ausführung umfasst das Hauptlager einen kugelkalottenförmigen Aufnahmesitz, in dem eine Fixierhülse schwenkbar gehaltert ist. Durch die kugelartige Grenzfläche zwischen Fixierhülse und Aufnahmesitz sind rotatorische Freiheitsgrade in allen Richtungen gegeben. Besonders günstig ist diese Konstruktion, wenn eine transfacettale Schraube zur Fusion vorgesehen ist.

Das Fusionsmodul ist aus Gründen erhöhter Befestigungssicherheit in der Regel starr an dem Tragmodul angeordnet. Es soll aber nicht ausgeschlossen sein, dass es flexibel an dem Tragmodul angeordnet ist. Dies kann Vorzüge hinsichtlich der Kompatibilität mit dynamischen Bewegungen der Wirbelsäule mit sich bringen.

Die Anlagefläche der Backenelemente kann so geformt sein, dass sie im cephaladen Bereich schmaler ist. Damit kann die Stärke des Implantats in einem kritischen Bereich, in dem es ansonsten leicht zu einer unerwünschten Druckausübung auf im Spinalkanal laufende Nervenstränge oder diese umgebendes Gewebe kommen könnte, verringert werden. Die Verträglichkeit des Implantats erhöht sich damit. Mit Vorteil ist das Fusionsmodul im caudalen Bereich der Anlageflächen angeordnet. Hier steht ausreichend Bauraum für das Implantat zur Verfügung, ohne dass eine erhöhte Gefahr von Irritationen von umgebendem Gewebe geschaffen wird.

In vielen Fällen ist bei einem Wirbel, an dem das erfindungsgemäße Fusionsimplantat angeordnet werden soll, die Lamina wegen einer Dekompressionsbehandlung vollständig entfernt. Über das in den Freiraum eingesetzten Tragmodul werden die Resektionsflächen der Lamina miteinander verbunden, so dass funktional der Bogen wieder geschlossen ist. Es kann aber auch sein, dass eine vollständige Resektion der Lamina über ihre volle Höhe nicht erforderlich ist, sondern dass sie über einen Teil ihrer Höhe bestehen bleibt. Dieser Rest alleine ist mechanisch nicht mehr voll belastbar. Hier setzt die Erfindung mit einer Variante des Fusionsimplantats an, die eine geringe Bauhöhe hat. Das zur Einbettung in die Lamina vorgesehene Tragmodul des Fusionsimplantats weist dazu eine geringere Abmessung in cephalad-caudaler Richtung auf, die vorzugsweise weniger als die Hälfte der Erstreckung der Anlagefläche in dieser Richtung beträgt. Damit ist das Fusionsimplantat so kompakt, dass es unter den Laminarest gesetzt werden und ihn verstärken kann, wobei es gleichzeitig eine solide Basis für die Facettenfusion bildet. Vorzugsweise ist das Fusionsmodul im mittleren Bereich der Backenelemente angeordnet, und die Backenelemente sind gegeneinander tauschbar. Damit kann die Relation zwischen Backenelementen und ihrer Führung invertiert werden. Bildet in der Grundversion die Führung mit den Backenelementen eine nach unten offene U- bzw. H-förmige Struktur, so entsteht mit der Inversion dank der Tauschbarkeit der Backenelemente eine nach oben offene U-Form. Diese eignet sich besonders zur Implantation unter einem Resektionsrest der Lamina mit dem spinalen Vorsprung.

Das Tragmodul ist vorzugsweise insbesondere mit seiner Führung so auf das Fusionsmodul abgestimmt, dass ein koaxialer Zugangsweg zur transfacettalen Schraube im Fusionsmodul frei ist. Mit anderen Worten, es besteht ein ungehinderter Zugang zum Kopf der transfacettalen Schraube in Richtung der verlängerten Schraubenachse. Damit ist sind die konstruktiven Voraussetzungen geschaffen, um die transfacettale Schraube im montierten Zustand am Implantationsort festziehen zu können.

Bei einer Ausführungsform ist eine Pedikelstütze an dem Tragmodul angeordnet. Diese besonders vorteilhafte Ausführungsform verdient gegebenenfalls unabhängigen Schutz auch ohne das Fusionsmodul. Die Pedikelstütze erlaubt eine sichere und robuste Fixierung, stellt aber hohe Ansprüche an die Positionierungsgenauigkeit. Dank der erfindungsgemäßen Anordnung an dem Tragmodul ist eine präzise Positionierung erreicht. so dass das im Stand der Technik vorhandene Risiko einer Fehlpositionierung, die zu beträchtlichen Schädigungen führen kann, eliminiert ist. Das gilt insbesondere dann, wenn an der Pedikelstütze ein Lager für Pedikelschrauben vorgesehen ist. Um dennoch eine ausreichende Anpassmöglichkeit an die individuelle Anatomie des Patienten zu ermöglichen, ist die Pedikelschraube vorzugsweise polyaxial gelagert. Dazu ist das Lager entsprechend dem der transfacettalen Schraube ausgebildet.

Die Pedikelstütze ist mit Vorteil an einer Ausrichteinrichtung des Tragmoduls gehaltert. An dieser Stelle kann die Pedikelstütze modular hinzugefügt oder weggelassen werden. Auch eine einseitige Anordnung der Pedikelstütze ist auf diese Weise leicht möglich.

Bei einer zweckmäßigen Ausführungsform ist die Pedikelstütze so ausgeführt, dass die Pedikelschrauben parallele Achsen aufweisen, die etwa quer zur Verstellrichtung der Führung liegt. Dies ermöglicht eine kompakte Ausführung der Pedkelstütze. Vorzugsweise weist sie eine solche Abmessung auf, dass ihre lateralen Abmessungen nicht mehr als das 1,5-fache der lateralen Abmessung des Tragmoduls ist. Alternativ kann die Pedikelstütze auch lateral ausgreifend vorgesehen sein, wobei sie deutlich weiter nach lateral überragt. Hierbei sind die Achsen der Pedikelschraube zur Mitte des Tragmoduls hin konvergierend ausgerichtet. Dies ermöglicht eine besonders stabile Befestigung.

Die Erfindung erreicht Befestigungssicherheit durch elastische Aufweitung der Lamina bzw. des Wirbelbogens. In Kombination mit den außen gelegenen Anlageflächen der Backenelemente hat dies den Vorteil, dass ein Kollabieren des Wirbelbogens, wie es bisher in unerwünschter Weise geschehen konnte, unmöglich gemacht ist. Im Gegenteil, unter dem bisher zum Kollabieren führenden Druck wird das Verstärkungsimplantat nur fester in seinen Sitz gepresst und kann damit seine Aufgabe erfüllen. Obgleich die elastische Aufweitung einen langzeitstabilen Sitz an sich gewährleisten kann, kann zusätzlich eine Rücklaufsperre für die Backenelemente vorgesehen sein, um so die langfristige Befestigungssicherheit weiter zu erhöhen.

Die Rücklaufsperre ist mit Vorteil als eine zwischen den Backenelementen und der Führung wirkende Ausrichteinrichtung ausgebildet. Mit einer solchen Klemmung lässt sich nach dem Expandieren auf einfache Weise die erreichte Expansionsposition fixieren. Damit wird ein Verrutschen der Backenelemente verhindert. Sollten die Anforderungen an die Sicherheit gegenüber unerwünschten Bewegungen der Backenelemente höher sein, so kann vorzugsweise die Rücklaufsperre mit Verrastungselementen versehen sein, die zwischen den Backenelementen und der Führung angeordnet sind. Zweckmäßigerweise umfassen die Verrastungsmittel eine Riffelung und in sie eingreifende Rastnasen. Mit einer solchen Verrastung, wie sie durch die Riffelung in Kombination mit der Rastnase erreicht ist, ergibt sich eine formschlüssige Sicherung. Dies bietet den Vorteil, auch bei sehr aktiven Patienten mit entsprechender Belastung der Wirbelsäule eine ausreichend sichere Halterung des Verstärkungsimplantats zu erreichen.

Vorzugsweise ist mindestens eines der Backenelemente mit einer Ausrichteinrichtung versehen. Damit kann die Orientierung von dessen Außenfläche zu der Führung verändert werden. Dies ermöglicht eine feinere Anpassung des Fusionsimplantats an die tatsächlichen anatomischen Verhältnisse nach der Laminektomie.

Besonders bewährt hat sich eine Ausführung eines Lagers der Ausrichteinrichtung als Klemmschraube. Im ungespannten Zustand ermöglicht sie eine Verdrehung um ihre Mittelachse, während sie im gespannten Zustand die erreichte Orientierung sichert. Zweckmäßigerweise ist das andere Backenelement entsprechend ausgerüstet.

Es hat sich bewährt, die Ausrichteinrichtung mit einer Verdrehsicherung zu versehen. Sie ist dazu ausgebildet, den Winkel zu begrenzen, mit dem die Backenelemente relativ zum Expansionselement einnehmen können. Bewährt hat sich eine Begrenzung auf einen Verstellbereich von maximal 45 Grad, vorzugsweise maximal 30 Grad.

Die Anlageflächen an den Backenelementen weisen vorzugsweise spitze Hervorstehungen (Spikes) auf. Bewährte Formen für solche Spikes sind bspw. Kegelspitzen, Pyramiden, prismatische oder V-förmige Erhebungen. Damit kann eine sichere primäre Fixation erreicht werden. Um zusätzlich auch eine schnelle und sichere sekundäre Fixation zu erreichen, sind vorzugsweise die Anlageflächen mit einer knochenwachstumsfördernden Beschichtung versehen. Hierbei kann es sich insbesondere um Hydroxylapatit oder andere osteoinduktive Substanzen handeln.

Die Anlageflächen sind vorzugsweise so an den beiden Backenelementen angeordnet, dass sie miteinander fluchten. Hierunter wird verstanden, dass sie in Richtung des Verstellwegs des Expansionselements gesehen weder einen horizontalen noch vertikalen Offset aufweisen. Damit wird eine asymmetrische Kraftbeaufschlagung des Fusionsimplantats verhindert, so dass keine unerwünschten Drehmomente auf das Fusionsimplantat wirken, welche es aus seiner vorgesehenen Position zu drehen trachten.

Gegenstand der Erfindung ist ferner ein Satz umfassend ein Implantat wie vorstehend beschrieben und ein Instrumentarium, welches umfasst einen langgestreckten Führungsdraht, einen Führungsschaft, durch welchen der Führungsdraht steckbar ist, mit einem zur Aufnahme am Hauptlager des Fusionsmoduls ausgebildetem Ende, und einen kanülierten Schraubendreher. Nach dem teilweisen oder vollständigen Resezieren der Lamina wird das Implantat in den so geschaffenen Freiraum eingesetzt. Hierzu kann eine optionale Setzpinzette vorgesehen sein. Im nächsten Schritt wird mittels einer optionalen Spreizzange, die bei gesonderter Ausführung dank ihrer Abwinkelung gleichzeitig mit der Setzpinzette angesetzt sein kann, das Expansionselement des Implantats gespreizt. Es sei angemerkt, dass die Spreizzange zusammen mit der Setzpinzette als kombiniertes Instrument ausgeführt sein kann. Es wird dann der Führungsdraht durch den Führungsschaft geschoben, und zwar durch das Hauptlager zuerst in die obere Facette und weiter in die untere Facette. Damit wird der Implantationspfad für die transfacettale Schraube definiert. Danach wird der Führungsschaft entfernt und ein Gewebeschutzrohr aufgeschoben. Sein Innendurchmesser ist so groß bemessen, dass die transfacettale Schraube hindurch geführt werden kann. Die transfacettale Schraube ist kanüliert, das heißt sie weist eine Durchgangsöffnung längs ihrer Mittelachse auf, und kann so auf den Führungsdraht aufgeschoben und auf diesem durch das Gewebeschutzrohr an das Fusionsmodul geführt werden. Mittels eines ebenfalls kanülierten Schraubendrehers, der auf den Führungsdraht in der gleichen Weise aufgesteckt ist, wird die transfacettale Schraube festgeschraubt. Hierbei bleibt dank des Führungsdrahts die Position kontrolliert. Ist die Schraube festgezogen, ist die Position fixiert und der Führungsdraht mit dem Gewebeschutzrohr kann entfernt werden. Nachdem die transfacettale Schraube auf der anderen Seite auf dieselbe Weise befestigt ist, kann die Spreizzange entfernt werden. Es sei angemerkt, dass auch solide transfacettale Schrauben eingesetzt werden können, die dann aber beim Einsetzen nicht durch den Führungsdraht geführt sind. Abschließend können die Klemmschrauben festgezogen werden und das Expansionselement des Fusionsimplants damit festgesetzt werden. Das Implantat ist damit eingesetzt.

Die Erfindung erstreckt sich weiter auf ein Verfahren zur Implantation, das mit den vorgenannten Schritten durchgeführt wird. Es eignet sich insbesondere zur Implantation mit einem wenig invasiven dorsalen Zugang ("limited invasive dorsal approach").

Die Erfindung wird nachfolgend in Bezugnahme auf die beigefügten Zeichnungen anhand von vorteilhaften Ausführungsbeispielen erläutert. Es zeigen:
- Fig. 1 a, b: eine perspektivische Ansicht eines ersten Ausführungsbeispiels in demontiertem und montiertem Zustand;

- Fig. 2 a, b: eine Seitenansicht sowie eine Aufsicht des Ausführungsbeispiels gemäß Figur 1;
- Fig. 3: eine Detailvergrößerung zu dem in Figur 1 dargestellten Ausführungsbeispiel;
- Fig. 4 a, b: eine perspektivische Ansicht eines zweiten Ausführungsbeispiels in demontiertem und montiertem Zustand;
- Fig. 5: eine Aufsicht auf das in Figur 4 dargestellte zweite Ausführungsbeispiel;
- Fig. 6: eine Detailvergrößerung der zu dem in Figur 4 dargestellten zweiten Ausführungsbeispiel;
- Fig. 7: eine Variante des zweiten Ausführungsbeispiels;
- Fig. 8: eine Explosionsansicht eines dritten Ausführungsbeispiels;
- Fig. 9: Detailansichten zu Befestigungsschrauben des in Figur 7 dargestellten dritten Ausführungsbeispiels;
- Fig. 10 a, b: Varianten zu dem in Figur 8 dargestellten dritten Ausführungsbeispiel;
- Fig. 11: ein Instrumentarium zur Implantation in Implantaten gemäß den Ausführungsbeispielen;

- Fig. 12: ein Verwendungsbeispiel für Instrumente aus dem Instrumentarium;
- Fig. 13 a - d: Darstellungen zu Arbeitsschritten bei der Implantation; und
- Fig. 14 a - c: Darstellungen von Implantaten gemäß den Ausführungsbeispielen in implantiertem Zustand.

Ein erstes Ausführungsbeispiel für ein erfindungsgemäßes Fusionsimplantat umfasst in seiner Gesamtheit mit der Bezugsziffer 1 bezeichnetes Tragmodul und ein Fusionsmodul 6. Das Tragmodul 1 ist in Stegbauweise ausgeführt. Es umfasst einen Kulissenkörper 2, in dem ein Schlitten 3 längsverschieblich geführt ist. An dem Kulissenkörper 2 sowie an dem Schlitten 3 ist jeweils ein Backenelement 4, 4' angeordnet. Sie weisen an ihrem jeweils nach außen, d.h. voneinander wegweisenden Seiten eine Anlagefläche 43 an die Lamina eines Wirbels auf.

Der Kulissenkörper 2 und der Schlitten 3 wirken derart zusammen, dass sich bei der Längsverschiebung der Abstand zwischen den Backenelementen 4, 4' ändert. Der Kulissenkörper 2 und der Schlitten 3 wirken daher nach Art eines Expansionselements zusammen, welches den Abstand zwischen den beiden Außenflächen 43 der beiden Backenelemente 4, 4' verschieden groß einstellen kann. Je nach Einstellung des Expansionselements, d.h. relativer Positionierung zwischen dem Kulissenkörper 2 und dem längsverschieblich daran geführten Schlitten 3 kann ein Tragmodul 1 geschaffen werden, welches unterschiedlich breite, durch Resektion entstandene Zwischenräume in der Lamina 93 eines Wirbelkörpers überbrücken kann.

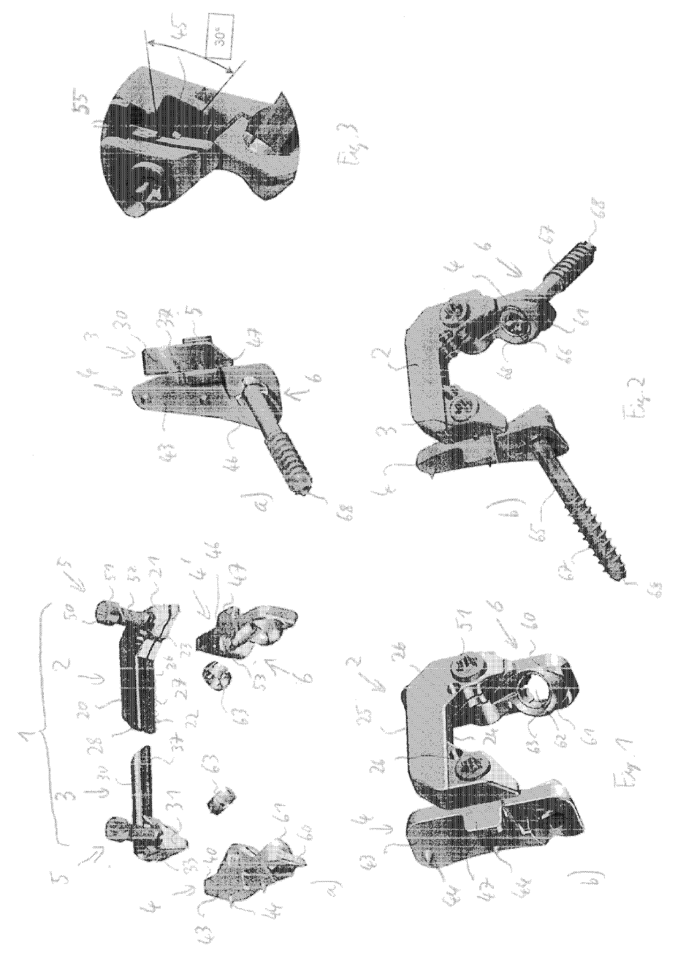

Der Kulissenkörper 2 weist einen Tragsteg 20 auf, an dessen einem (in Figur 1 rechten) Ende ein Halter 21 ausgebildet ist. Der Steg 2 ist im Querschnitt rechteckförmig und weist zwei Lateralseiten 24, 25 auf. Parallel zu den Lateralseiten 24, 25 ist in dem Steg 20 eine bei dem dargestellten Ausführungsbeispiel kreisförmig ausgebildete Führungsnut 28 angeordnet. Sie ist über einen Schlitz 27 mit der Lateralseite 24 des Stegs 20 verbunden, wobei sich der Schlitz 27 bis in den Halter 21 erstreckt. Der Übergangsbereich zwischen der Lateralseite 25 des Stegs 20 und dem Halter 21 an besagtem Ende des Stegs 20 kann mit einer Abschrägung versehen sein.

Der Schlitten 3 weist als Hauptkomponenten einen Führungsstab 30 und einen Halter 31 auf, der hinsichtlich seiner Außenform spiegelsymmetrisch zu dem Halter 21 des Kulissenkörpers 2 ausgeführt ist. Der Führungsstab 30 weist eine zu der Nut 28 des Kulissenkörpers 2 komplementäre Form auf, so dass eine längsverschiebliche Führung gebildet ist. In dargestelltem Ausführungsbeispiel ist wegen der kreisrunden Form der Führungsnut 28 der Führungsstab 30 mit einem kreisförmigen Querschnitt versehen. Zur Erzielung einer Verdrehsicherheit ist an der zu dem Halter 31 gewandten Seite des Führungsstabs 30 eine schmale Führungsleiste 37 ausgebildet. Ihre Abmessungen sind so gewählt, dass sie schmaler ist als die Weite des Schlitzes 27 am Kulissenkörper 2 in einem entspannten Zustand, aber mindestens so groß wie die Weite in einem gespannten Zustand, wie später noch näher erläutert werden wird. Damit ist der Schlitten 3 längsverschieblich, aber rotationsgesichert an dem Kulissenkörper 2 geführt.

Die Halter 21, 31 an dem Kulissenkörper 2 beziehungsweise dem Schlitten 3 sind im Wesentlichen spiegelsymmetrisch aufgebaut. Sie werden daher nachfolgend gemeinsam erläutert. Die Halter 21, 31 umfassen jeweils eine Durchgangsöffnung 23 beziehungsweise 33, deren Achse lotrecht zu der Achse der längsverschieblichen Bewegung zwischen dem Kulissenkörper 2 und dem Schlitten 3 orientiert ist. Die Durchgangsöffnungen 23, 33 dienen einer Ausrichteinrichtung 5 zum Positionieren und Sichern jeweils eines der Backenelemente 4, 4'.

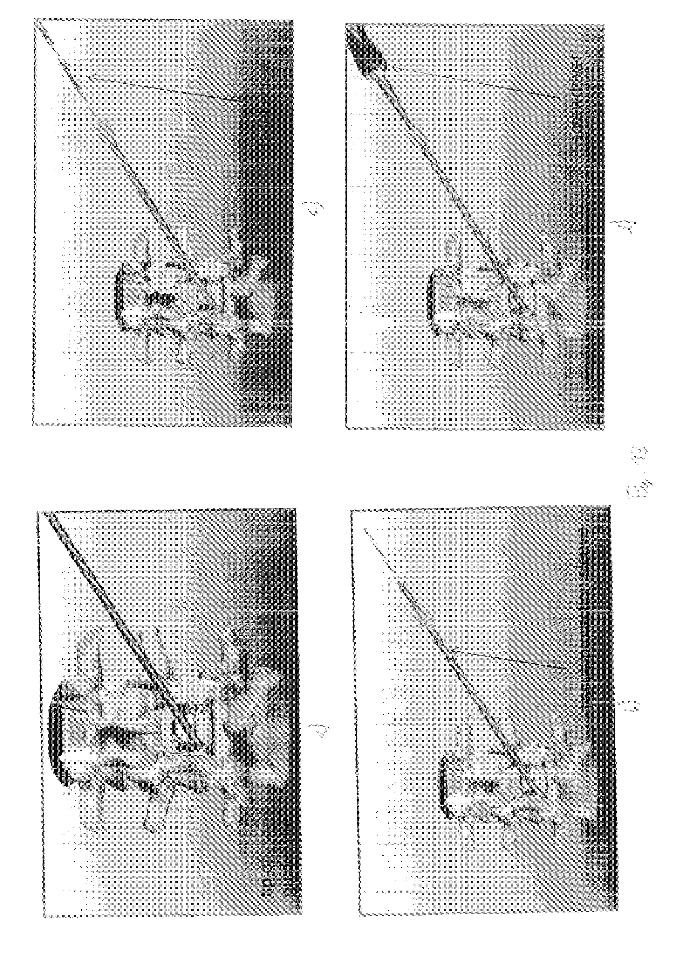

Die Backenelemente 4, 4' sind ebenfalls spiegelsymmetrisch zueinander aufgebaut. Sie weisen an ihrer jeweils von dem anderen Backenelement 4', 4 wegweisenden Anlagefläche 43 eine Mehrzahl von Spikes 44 zur Verankerung in lateralen Schnittflächen 94 der Lamina 93 des Wirbels 9 auf (siehe Figur 13). Die Anlageflächen 43 mit den Spikes 44 sind vorzugsweise mit einer knochenwachstumsfördernden Beschichtung, wie Calciumphosphat oder Hydroxylapatit versehen. Am Übergang der Anlageflächen 43 zur Oberseite der Backenelemente 4, 4' ist jeweils eine nach außen ragende Nase 47 angeordnet. Sie fungiert als Tiefenanschlag und begrenzt die Einschubtiefe der Backenelemente 4, 4' und damit des gesamten Implantats an der Lamina 93. Ein versehentliches zu weites Einschieben, welches zu Irritationen oder Verletzungen von im Markkanal befindlichen Gewebe- oder Nervensträngen führen könnte, wird damit vermieden. Weiter ist an der Oberseite der Backenelemente 4, 4' jeweils ein Rastvorsprung 46 angeordnet. Er ist dazu ausgebildet, mit einer an der Unterseite 22 des Stegs 20 angeordneten Riffelung 26 zusammenzuwirken. Damit soll erreicht werden, dass im montierten Zustand die Backenelemente 4, 4' gegenüber einem unbeabsichtigten Verdrehen sowie Verschiebung im Fall des Backenelements 4' gegenüber dem Steg 20 gesichert sind. Der mit der Riffelung 26 zusammenwirkende Rastvorsprung 46 bildet eine Verrastung, welche die Position der Backenelemente 4, 4' formschlüssig sichert, so dass eine unbeabsichtigte Verstellung auch bei hoher Belastung ausgeschlossen ist.

Zum Arretieren der Position der Backenelemente 4, 4' gegenüber dem Steg 20 sind Ausrichteinrichtungen 5, 5' an den Haltern 21, 31 vorgesehen. Sie weisen jeweils eine in ihrer Gesamtheit mit der Bezugsziffer 50 bezeichnete Klemmschraube auf, welche einen Schraubenkopf 51 und einen Schaft 52 mit einem Außengewinde aufweist. Der Kopf 51 weist einen größeren Durchmesser als die Durchgangsöffnung 23, 33 in dem Halter 21 beziehungsweise 31 auf, so dass der Schaft 52 durch sie hindurch in ein entsprechendes Gegengewinde in dem Backenelement 4, 4' greifen kann. Durch Festziehen der Klemmschraube 50 wird somit das Backenelement 4, 4' gegen die Unterseite des aus dem Kulissenkörper 2 und dem Schlitten 3 gebildeten Expansionselements gezogen, wodurch die expandierte Position der Backenelemente sowohl hinsichtlich ihres relativen Abstandes voneinander wie auch in der relativen Positionierung (auch in Bezug auf den Steg 20, sowohl kraft- wie auch formschlüssig fixiert ist).

Unter der Wirkung der Ausrichteinrichtung 5 ist ebenfalls der Schlitz 27 des Kulissenkörpers 2 zusammengedrückt, und zwar soweit, dass er die Führungsleiste 37 einklemmt. Damit wird eine weitere Sicherung des Schlittens 3 gegenüber einer unerwünschten Längsverschiebung relativ zu dem Kulissenkörper 2 erreicht.

Die Anlagefläche 43 der Backenelemente 4, 4' sind im Wesentlichen in eine Richtung parallel zu den Außenflächen der Halter 21, 31 länglich ausgestreckt. Dennoch ist die Tiefe der Elemente 4, 4' an einem cephaladen, stegnäheren Ende dünner ausgeführt als an dem gegenüberliegenden caudalen, stegfernen Ende. An diesem ist ein Fusionsmodul 6 angeordnet, welches nachfolgend näher beschrieben wird.

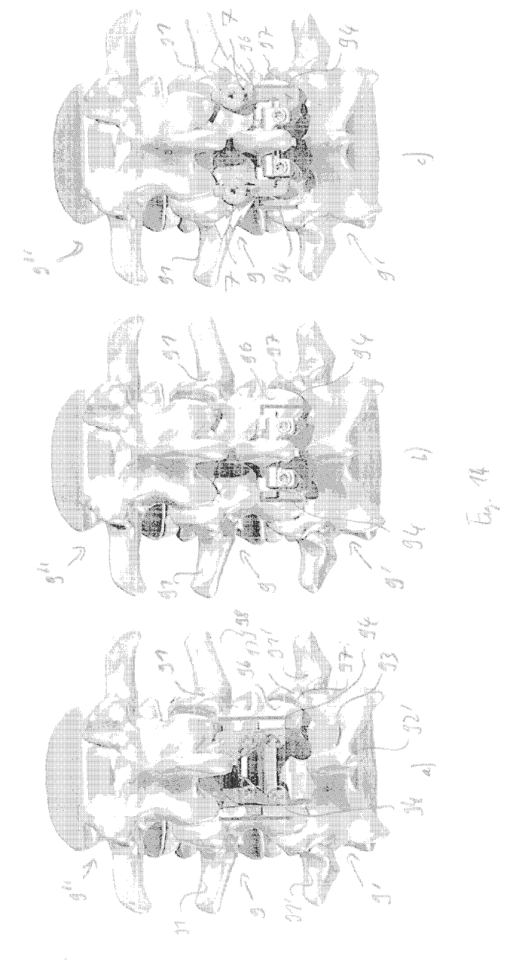

Das Fusionsmodul 6 dient dazu, das untere Facettengelenk des Wirbels 9, an dem das Implantat angeordnet ist, mit den oberen Facetten des darunter angeordneten Wirbels 9' zu fusionieren (siehe insbesondere Figur 14). Dazu weist das Fusionsmodul 6 ein Hauptlager an einer oberen Facette 96 und ein Gegenlager an einer unteren Facette 97 des Facettengelenks 98 auf. Das Hauptlager 60 ist an dem stegfernen Ende der Backenelemente 4, 4' angeordnet. Es umfasst einen Aufnahmesitz 61, der eine Öffnung 62 mit einem kugelkalottenförmig ausgebildeten Innenraum aufweist. Darin ist eine Fixierhülse 63 angeordnet, deren Außenmantel kugelförmig geformt ist. Sie ist damit schwenkbar in dem Aufnahmesitz 61 gehaltert. Durch die Fixierhülse 63 ist eine transfacettale Schraube 65 gesteckt, die einen Kopf 66 an ihrem proximalen Ende und ein Gewinde 67 in ihrem distalen Bereich aufweist. Die Länge der Schraube 65 ist so gewählt, dass sie etwa der Dicke der beiden Facetten 96, 97 entspricht, die gemeinsam das Facettengelenk 98 bilden. Die Schraube 65 weist längs ihrer Mittelachse eine Durchgangsöffnung 68 auf ("kanülierte Schraube"). Die schwenkbewegliche Lagerung der Fixierhülse 63 in dem Aufnahmesitz 61 ermöglicht es, dass die Schraube 65 in verschiedener Achsorientierung gehaltert sein kann. Im dargestellten Ausführungsbeispiel kann die Schraube bezogen auf die Mittelachse der Öffnung 62 um einen Winkel von ± 15° in alle Richtungen verschwenkt werden. Bei erhöhter Baugröße sind auch größere Verschwenkwinkel möglich, insbesondere bis zu 20° oder 25°. Die transfacettale Schraube 65 dient dazu, nach dem Einsetzen des Tragmoduls 1 eingeschraubt zu werden und mit ihren in die Facette 97 des benachbarten Wirbelkörpers 9' eingreifenden Gewinde 67 ein Gegenlager zu dem an dem Fusionsmodul 6 gebildeten Hauptlager zu bilden, um so beim Einziehen der Schraube die Facetten 96, 97 fest gegeneinander zu ziehen und das Facettengelenk zu immobilisieren. Die transfacettale Schraube 65 kann an ihrem Schaft und/oder Gewinde 67 eine knochenwachstumsfördernde Beschichtung aufweisen.

Die Backenelemente 4, 4' sind um die Achse der Schrauben 50 schwenkbeweglich an den Haltern 21, 31 gehaltert. Um den Winkelbereich auf ein praktikables Maß zu begrenzen, um so insbesondere eine unerwünschte Verdrehung der Backenelemente 4, 4' gerade beim Einsetzen des Tragmoduls 1 an seinen vorgesehenen Implantationsort zu wirken, ist vorzugsweise eine Winkelbegrenzungseinrichtung vorgesehen. Sie ist bei dem dargestellten Ausführungsbeispiel gebildet von einem drehfest an dem Backenelement 4, 4' angeordneten Radialvorsprung 45, der zwischen Seitenwänden einer Ausnehmung 55 an der Unterseite der Halter 21, 31 geführt ist. Hierbei wirken die Seitenwände der Ausnehmung 55 als Anschläge, welche den Schwenkwinkel der Backenelemente 4, 4' relativ zu den Haltern 21, 31 begrenzt sind. In dem dargestellten Ausführungsbeispiel sind die Abmessungen der Ausnehmung 55 so gewählt, dass sich ein Schwenkwinkel von insgesamt 30° für jedes der Backenelemente 4, 4' ergibt.

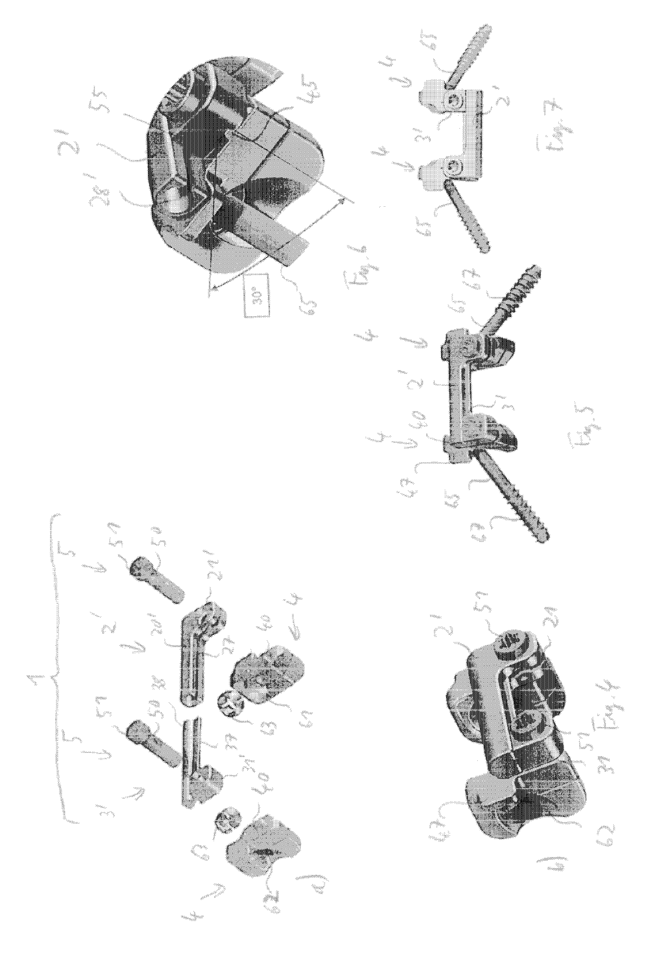

In Figur 4a, b ist ein zweites Ausführungsbeispiel dargestellt. Dieses unterscheidet vom ersten Ausführungsbeispiel gemäß den Figuren 1 bis 3 im Wesentlichen dadurch, dass das Tragmodul eine deutlich geringere Bauhöhe aufweist. Die Halter 21', 31' sind kompakter ausgeführt als die Halter 21, 31 bei dem ersten Ausführungsbeispiel. Weiter ist der Tragsteg 20' im Querschnitt nicht rechteckförmig, sondern quadratisch ausgeführt. Damit erfordert das aus dem Kulissenkörper 2' in dem Schlitten 3' gebildete Tragmodul 1' eine niedrigere Bauhöhe im Vergleich zu dem Tragmodul 1 des ersten Ausführungsbeispiels. Dies ermöglicht es, das Implantat in eine Teilresektion der Lamina zu implantieren. Dadurch kann eine Laminabrücke bestehen bleiben, so dass zum einen die natürliche Stabilität des Wirbelkörpers weitgehend erhalten bleibt und zum anderen der nach hinten abragende spinale Vorsprung an dem Wirbel erhalten bleiben kann. Die Implantation ist weniger invasiv und schonender für den Patienten. Bei diesem zweiten Ausführungsbeispiel ist, wie aus Figur 4 ersichtlich, das Fusionsmodul 6 nicht am Rand der Backenelemente 4, 4' angeordnet, sondern vielmehr etwa in deren Mitte. Dies ermöglicht eine kleinere Ausführung der Backenelemente 4, 4'. Im Übrigen ist das zweite Ausführungsbeispiel ähnlich zu dem ersten Ausführungsbeispiel ausgeführt, so dass auf vorstehende Erläuterungen verwiesen werden kann.

Eine Variante des zweiten Ausführungsbeispiels ist in Figur 7 dargestellt. Hierbei sind der Kulissenkörper 2' und der Schlitten 3' mit inverser Lage an den Backenelementen 4, 4' angeordnet. Das Tragmodul 1' bildet daher eine deutliche U-Form, wobei das U in Bezug auf die Implantationslage nach oben offen ist. Bei der Grundform, wie in Fig. 4a abgebildet, liegt eher eine H-Form bzw. eine nach unten offene U-Form vor.

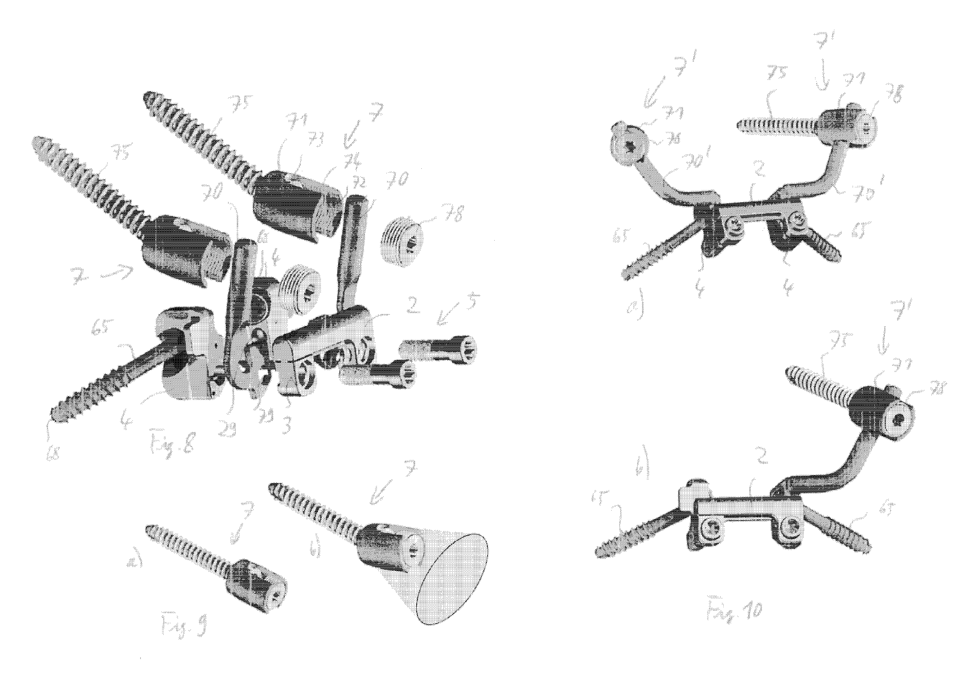

Ein drittes Ausführungsbeispiel ist in Figur 8 dargestellt. Es basiert auf dem in Figur 4 bis 6 dargestellten zweiten Ausführungsbeispiel und unterscheidet sich von diesem im Wesentlichen dadurch, dass eine zusätzliche Pedikelstütze 7 vorgesehen ist. Die Pedikelstütze 7 stellt eine zusätzliche Befestigungsmöglichkeit des Implantats an dem Wirbelkörper dar. Sie erhöht die Stabilität. Die Pedikelstütze 7 umfasst jeweils eine Pedikelschraube 75 für den linken und den rechten Pedikel 91 des Wirbelkörpers 9. Nachstehend wird der Aufbau in Bezug auf die rechte Pedikelschraube 75 erläutert; für die linke Pedikelschraube 75 gilt entsprechendes. Die Pedikelschraubenstütze 7 ist über einen Tragstab 70 an dem Tragmodul 1 angeordnet. Der Tragstab 70 weist an seinem unteren Ende einen plattenförmigen Fortsatz auf, in dem eine Durchgangsöffnung 79 ausgebildet ist. Der plattenförmige Fortsatz liegt mit seiner Unterseite auf der Oberseite des Backenelements 4 an und mit seiner Oberseite liegt er an der Unterseite des Kulissenkörpers 2 an. Die Schraube 50 in der Ausrichteinrichtung 5 ist durch die Durchgangsöffnung 79 in dem Fortsatz geführt. Damit wird beim Festziehen des Backenelements 4 an dem Kulissenkörper 2 auch der Tragstab 70 befestigt. An seinem gegenüberliegenden Ende ist der Tragstab 70 kreisförmig ausgebildet.

Es sind eine Hülse 71, ein Spannkäfig 73 sowie ein Druckelement 78 vorgesehen. Die Hülse 71 ist hohlzylinderartig ausgeführt mit einer von einem hinteren zu einem vorderen Ende hin durchlaufenden Öffnung 72. Sie weist in ihrem hinteren Bereich ein Innengewinde und in ihrem vorderen Bereich einen kugelkalottenförmig ausgebildeten Passsitz für den Spannkäfig 73 auf. Der Spannkäfig 73 ist ähnlich zu der Fixierhülse 63 ausgeführt und weist vorzugsweise dieselben Abmessungen auf. Der Kerndurchmesser des Innengewindes ist so gewählt, dass der Spannkäfig 73 durch das Innengewinde hindurch an seinen Passsitz geschoben werden kann. Die Hülse 71 weist weiter zwei diametral gegenüberliegende Längsschlitze 74 auf. Sie erstrecken sich vom hinteren Ende der Hülse 71 über den gesamten Bereich des Innengewindes bis in den Bereich des Passsitzes der Durchgangsöffnung 72. Der Spannkäfig 73 ist genauso schwenkbar in seinem Passssitz gelagert wie die Fixierhülse 63 in dem Aufnahmesitz 61 des Fusionsmoduls 6.

Im Spannkäfig 73 ist die Pedikelschraube 75 eingesteckt, und zwar derart, dass sie mit ihrem Kopf in den Spannkäfig aufgenommen ist. Der in den Schlitz 74 gesteckte Tragstab 70 drückt dabei auf den Kopf der Pedikelschraube 75. Indem die Madenschraube 78 in das Innengewinde der Durchgangsöffnung 72 soweit eingeschraubt wird, bis die Madenschraube 78 an dem Tragstab 70 anschlägt, kann durch weiteres Einschrauben der Tragstab 70 gegen den Kopf der Schraube 75, und dieser wiederum über den Spannkäfig 73 so verspannt werden, dass die Pedikelschraube 75 in ihrer axialen Orientierung zur Hülse 71 festgeklemmt ist. Damit ist eine polyaxiale Lagerung der Pedikelschraube erreicht, und zwar in einem Winkelbereich von ± 15° um die Mittelachse der Hülse 71 (siehe Figur 9b). Die schwenkbare Ausführung ist nicht zwingend, es kann auch vorgesehen sein, dass die Pedikelschraube 75 sich in linearer Fortsetzung der Achse der Hülse 71 befindet, wie in Figur 9a dargestellt. Durch Einschrauben der Pedikelschraube 75 in den Pedikel 91 des Wirbels 9 und anschließendem Verspannen mittels der Madenschraube 78 kann damit eine zusätzliche Befestigung erreicht werden.

Bei einer Variante des dritten Ausführungsbeispiels ist der Tragstab bogenförmig ausgeführt und lädt weit nach lateral aus (siehe Figur 10a). Damit kann ebenfalls eine Pedikelschraubenfixierung erreicht werden, die jedoch im Gegensatz zu der in Figur 9a, b dargestellten Variante eine laterale Anordnung der Pedikelschraube 75 vorsieht. Die in Figur 9a, b dargestellte Variante bewirkt hingegen eine mediale Anordnung der Pedikelschrauben. Die laterale Anordnung kann den Vorteil für sich buchen, eine Abstützung auf einer breiteren Basis zu schaffen, hat jedoch den mitunter beträchtlichen Nachteil, dass sie wegen ihrer ausladenden Konstruktion zu einer erhöhten Irritation des umliegenden Gewebes führt. Es ist nicht zwingend erforderlich, dass die laterale Abstützung auf beiden Seiten erfolgt. Es kann bei weiteren Varianten auch vorgesehen sein, dass der Tragstab einstückig mit dem Backenelement ausgeführt ist und/oder dass die Pedikelschraube lediglich einseitig vorgesehen ist (siehe Figur 10b).

Nachfolgend wird das zur Implantation vorgesehene Instrumentarium beschrieben. Es umfasst einen Führungsdraht 80, einen Führungsschaft 81, ein Gewebeschutzrohr 82, einen kanülierten Schraubendreher 83, einen weiteren Schraubendreher 84, eine Pinzette 85 sowie eine Spreizzange 87. Die Implantation erfolgt bei einem Fusionsimplantat gemäß dem ersten Ausführungsbeispiel in der folgenden Weise. Zuerst wird mittels eines geeigneten Resektionsinstruments (nicht dargestellt), wie es an sich aus dem Stand der Technik bekannt ist, eine für das erste Ausführungsbeispiel vollständige Resektion des rückwärtigen Teils der Lamina mit dem spinalen Vorsprung. Es werden damit zwei laterale Laminaresektionsflächen 94 gebildet. Zwischen ihnen entsteht ein Freiraum, der Zugang zu dem Kanal gibt. Es kann nun auf an sich bekannte Weise eine Dekompression durchgeführt werden. Ist dies erfolgt, wird das Implantat gemäß dem ersten Ausführungsbeispiel eingesetzt. Es wird dazu an seinen Ort gesetzt mittels der Setzpinzette 85. Dies geschieht in der Weise, in dem Aufnahmespitzen 86 der Setzpinzette 85 in Aufnahmeöffnungen 26 an der Oberseite des Tragmoduls 1 eingesteckt werden. Die Aufnahmeöffnungen 26 können wie in Figur 1 dargestellt, gesonderte Öffnungen sein, oder es kann sich wie in Figur 2 dargestellt, um eine Kombination mit ohnehin vorhandenen Öffnungen zur Werkzeugaufnahme am Schraubenkopf 51 der Ausrichteinrichtung 5 handeln. Die Aufnahme des Implantats an der Setzpinzette 85 erfolgt über Reibschluss.

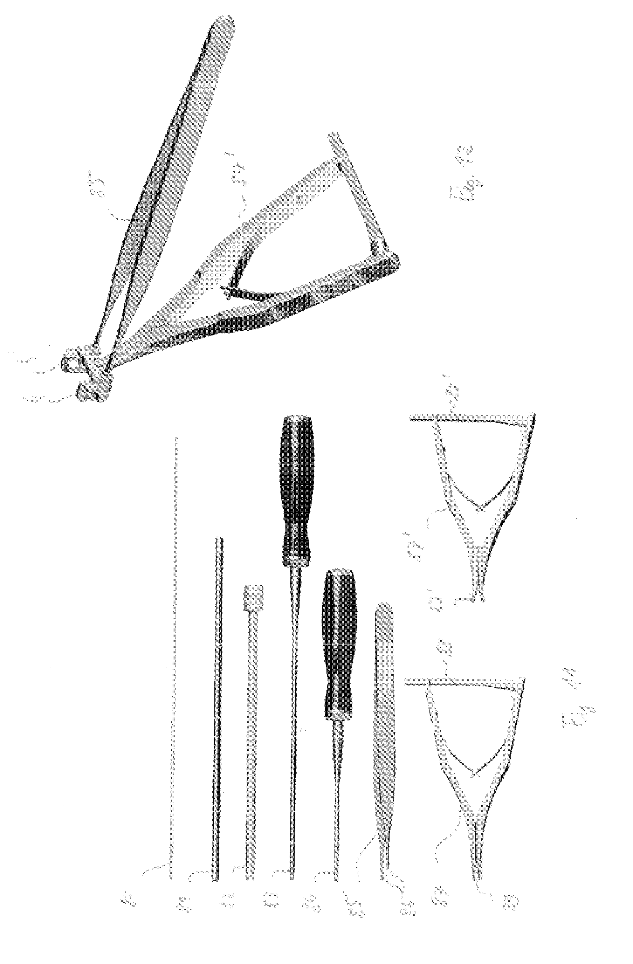

Das Implantat wird an seinen vorgesehenen Implantationsort im Freiraum zwischen den Laminaresektionsflächen 94 geführt und mittels einer Spreizzange 87 gespreizt. Dadurch entfernt sich der Schlitten 3 von dem Kulissenkörper 2 derart, dass die Backenelemente 4, 4' sich soweit auseinander bewegen, bis sie mit ihren Außenflächen 43 an den LaminaResektionsflächen 94 anliegen. Dabei wird die Spreizzange 87 gesetzt, bevor die Setzpinzette 85 abgenommen wird. Dies geschieht auf die in Figur 12 dargestellte Weise, nämlich indem die Spreizzange 87 von unten zugeführt wird. Die Spreizzange 87 weist an ihrem vorderen Ende Greifelemente 89 auf, die an eine entsprechende Gegenfläche an der Innenseite der Backenelemente 4, 4' eingreifen. Die Greifelemente 89 können insbesondere als Greifkugeln 89' ausgeführt sein, welche in Greifmulden 29 (s. Fig. 9) an den Innenseiten der Backenelemente 4, 4' formschlüssig eingreifen. Ist die Spreizzange 87' wie in Figur 12 dargestellt in Eingriff mit den Backenelementen 4, 4' gebracht, so kann mittels der Ratsche 88' die Spreizstellung der Spreizzange fixiert werden. Die Setzpinzette 85 kann nun abgenommen werden. Das Implantat ist unter der Wirkung der Spreizzange 87' an seinem Ort gehaltert. Die Spreizzange 87' ist so nach cephalad abgewinkelt geformt, dass auch im angesetzten Zustand Zugang zu dem Fusionsmodul 6, und insbesondere die darin einzusetzende transfacettale Schraube 65 besteht. Damit ist eine Primärpositionierung erreicht.

Zum positionsgenauen Setzen der transfacettalen Schraube 65 wird der Führungsschaft 81 in der richtigen Orientierung in die Öffnung 62 des Fusionsmoduls 6 eingesetzt. Dies kann unter Röntgenkontrolle folgen. Ist der Führungsschaft 81 richtig positioniert, wird der Führungsdraht 80 durch den Schaft eingesetzt und durch die Facetten 96, 97 bewegt. Hat der Führungsdraht 80 seine Position erreicht, wird der Führungsschaft 81 durch ein Gewebeschutzrohr 82 ersetzt. Die kanülierte Facettenschraube 65 wird mit ihrer Hohlbohrung 68 auf dem Führungsdraht 80 aufgefädelt und mithilfe des gleichfalls kanülierten Schraubendrehers 83 durch das Gewebeschutzrohr 82 zu dem Fusionsmodul 6 geführt und eingeschraubt. Dank der Kanülierung ist ein Festziehen der Schraube mittels des Schraubendrehers 83 ermöglicht, wobei die Positionierung durch den Führungsdraht 80 gewährleistet ist. Ist die Schraube 65 festgedreht, kann der Schraubendreher 83 entnommen und der Führungsdraht 80 mit dem Gewebeschutzrohr 82 entnommen werden. Derselbe Vorgang wird für die kontralaterale Facettenschraube 65 auf der anderen Seiten durchgeführt. Sind beide Facettenschrauben 65 angezogen, kann die Ausrichteinrichtung 5, 5' durch Festziehen der Klemmschrauben 50 mit dem Schraubendreher 84 betätigt werden, und somit die Spreizposition des Tragmoduls 1 fixiert werden. Die Spreizzange 87' kann nunmehr abgenommen werden. Das Implantat ist an seinem Ort fixiert.

Die somit erreichte Einbauposition ist in Figur 14a für das Implantat gemäß dem ersten Ausführungsbeispiel dargestellt. Man erkennt, dass das Implantat Lamina des Wirbels 9 vollständig ersetzt. In Figur 13b ist das zweite Ausführungsbeispiel im implantierten Zustand dargestellt. Dieses Implantat hat eine geringere Bauhöhe und ermöglicht einen teilweisen Erhalt der Lamina, nämlich in ihrem oberen Bereich mit dem spinalen Vorsprung. Diese schonende Variante ist in Figur 14b dargestellt. Die Implantation des dritten Ausführungsbeispiels mit den Pedikelschrauben gemäß Figur 8 ist in Figur 14c dargestellt.

## Patentansprüche

1. Fusionsimplantat für ein Facettengelenk, umfassend ein Tragemodul (1) und ein Fusionsmodul (2), wobei das Fusionsmodul (2) an dem Tragmodul (1) angeordnet ist und einen Halter für transfacettale Befestigungsmittel (65) aufweist,
**dadurch gekennzeichnet, dass**
das Tragmodul (1) ein Expansionselement und Backenelemente (4, 4') umfasst, die an voneinander wegweisenden Außenseiten Anlageflächen (43) für die Lamina (93) aufweisen und die längsverschieblich an einer Führung so angeordnet sind, dass ein Abstand der Fusionsmodule (6) mittels des Expansionselements veränderlich ist.

2. Fusionsimplantat nach Anspruch 1, **dadurch gekennzeichnet, dass**
das Fusionsmodul (6) ein Hauptlager (60) an einer oberen Facette (96) und vorzugsweise ein Gegenlager an einer unteren Facette (97) aufweist.

3. Fusionsimplantat nach Anspruch 2, **dadurch gekennzeichnet, dass**
das Hauptlager (60) schwenkbar ist.

4. Fusionsimplantat nach Anspruch 3, **dadurch gekennzeichnet, dass**
das Hauptlager (60) eine in einem kugelkalottenförmigen Aufnahmesitz (61) schwenkbar gehalterte Fixierhülse (63) umfasst.

5. Fusionsimplantat nach einem der Ansprüche 1 bis 4, **dadurch gekennzeichnet, dass**
das Fusionsmodul (6) als Befestigungsmittel eine transfacettale Schraube (65) lagert.

6. Fusionsimplantat nach einem der vorangehenden Ansprüche, **dadurch gekennzeichnet, dass**
das Fusionsmodul (6) flexibel an dem Tragmodul (1) angeordnet ist.

7. Fusionsimplantat nach einem der vorangehenden Ansprüche, **dadurch gekennzeichnet, dass**
die Backenelemente (4, 4') eine im cephaladen Bereich schmalere Anlagefläche (43) als im caudalen Bereich aufweisen.

8. Fusionsimplantat nach einem der vorangehenden Ansprüche, **dadurch gekennzeichnet, dass**
das Fusionsmodul (6) im caudalen Bereich der Backenelemente (4, 4') angeordnet ist.

9. Fusionsimplantat nach einem der vorangehenden Ansprüche, **dadurch gekennzeichnet, dass**
das Tragmodul (1) in cephalad-caudaler Richtung eine Abmessung aufweist, die weniger als die Hälfte der Erstreckung der Anlagefläche (43) in dieser Richtung beträgt.

10. Fusionsimplantat nach Anspruch 9, **dadurch gekennzeichnet, dass**
das Fusionsmodul (6) im mittleren Bereich der Backenelemente (4, 4') angeordnet ist.

11. Fusionsimplantat nach einem der Ansprüche 5 bis 10,
**dadurch gekennzeichnet, dass**
ein koaxialer Zugangsweg zu der transfacettalen Schraube (65) frei vom Tragmodul (1) ist.

12. Fusionsimplantat nach einem der vorangehenden Ansprüche, **dadurch gekennzeichnet, dass**
eine Pedikelstütze (7) an dem Tragmodul (1) angeordnet ist.

13. Fusionsimplantat nach Anspruch 12, **dadurch gekennzeichnet, dass**
die Pedikelstütze mittels der Ausrichteinrichtung (5) gehaltert ist.

14. Fusionsimplantat nach Anspruch 12 oder 13, **dadurch gekennzeichnet, dass**
die Pedikelstütze (7) ein Lager für Pedikelschrauben (75) aufweist.

15. Fusionsimplantat nach Anspruch 14, **dadurch gekennzeichnet, dass**
die Pedikelschrauben (75) parallele Achsen aufweisen, die quer zur Führungsrichtung liegt.

16. Fusionsimplantat nach einem der Ansprüche 12 bis 15,
**dadurch gekennzeichnet, dass**
die lateralen Abmessungen der Pedikelstütze (7) maximal das 1,5-fache der lateralen Abmessungen des Tragmoduls (1) sind.

17. Fusionsimplantat nach Anspruch 12, 13 oder 14, **dadurch gekennzeichnet, dass**
die Pedikelstütze (7') nach lateral ausgreifend ist und eine Achse der Pedikelschrauben zur Mitte des Tragmoduls (1) hin konvergierend ausgerichtet ist.

18. Fusionsimplantat nach einem der Ansprüche 14 bis 17,
**dadurch gekennzeichnet, dass**
die Pedikelstütze (7) eine polyaxiale Lagerung für die Pedikelschraube (75) aufweist.

19. Fusionsimplantat nach einem der vorangehenden Ansprüche, **dadurch gekennzeichnet, dass**
mindestens eines der Backenelemente (4) mit einer Ausrichteinrichtung (5) zur veränderbaren Orientierung des Backenelements (4) zum Tragmodul (1) versehen ist.

20. Fusionsimplantat nach Anspruch 19, **dadurch gekennzeichnet, dass**
eine Klemmschraube (50) vorgesehen ist, die als Lager für die Ausrichteinrichtung (5) vorgesehen ist.

21. Fusionsimplantat nach Anspruch 19 oder 20, **dadurch gekennzeichnet, dass**
die Ausrichteinrichtung (5) eine Verdrehsicherung (45, 55) aufweist, die zum Begrenzen eines Verdrehwinkels auf einen vorgegebenen Wert ausgebildet ist.

22. Fusionsimplantat nach einem der vorangehenden Ansprüche, **dadurch gekennzeichnet, dass**
das zweite Backenelement (4') mit einer Ausrichteinrichtung (5') versehen ist.

23. Fusionsimplantat nach einem der vorangegangenen Ansprüche, **dadurch gekennzeichnet, dass**
die Führung einen Stab (30) an einem Schlitten (3) und eine komplementär geformte Nut (28) an einem Kulissenkörper (2) umfasst.

24. Fusionsimplantat nach Anspruch 23, **dadurch gekennzeichnet, dass**
der Stab (30) und die Nut (28) über eine in einen Schlitz formschlüssig eingreifende Leiste verdrehgesichert sind.

25. Fusionsimplantat nach einem der vorangehenden Ansprüche, **dadurch gekennzeichnet, dass**
die Anlagefläche (43) spitze Hervorstehungen (46) und/oder eine knochenwachstumsfördernde Beschichtung aufweist.

26. Satz umfassend ein Fusionsimplantat nach einem der Ansprüche 1 bis 25 und ein Instrumentarium, **dadurch gekennzeichnet, dass** das Instrumentarium umfasst:
- einen langgestreckten Führungsdraht (80),
- einen Führungsschaft (81), durch welchen der Führungsdraht (80) steckbar ist, mit einem zur Aufnahme am Hauptlager (60) des Fusionsmoduls (6) ausgebildetem Ende, und
- einen kanülierten Schraubendreher (83).

27. Satz nach Anspruch 26, **dadurch gekennzeichnet, dass** das Instrumentarium weiter umfasst:
- eine Spreizzange (87, 87'), die vorzugsweise in ihrer Arbeitsstellung nach cephalad abgewinkelt ist und einen freien Zugang für den Führungsschaft gibt, und
- vorzugsweise eine gesonderte Setzpinzette vorgesehen ist, die vorzugsweise über einen reibschlüssige Verbindung mit dem Implantat verbunden ist.

28. Verfahren zum Einsetzen eines Fusionsimplantat, das ein Tragemodul und ein Fusionsmodul umfasst, wobei das Fusionsmodul an dem Tragmodul angeordnet ist und einen Halter für ein transfacettales Befestigungselement aufweist, und das Tragmodul (1) ein Expansionselement und Backenelemente (4, 4') umfasst, die an voneinander wegweisenden Außenseiten Anlageflächen (43) für die Lamina (93) aufweisen und die längsverschieblich an einer Führung so angeordnet sind, dass ein Abstand der Fusionsmodule mittels des Expansionselements veränderlich ist,
**gekennzeichnet durch** die Schritte:
- Resektion zumindest eines Teils der Lamina eines Wirbelkörpers,
- Einsetzen des Fusionsimplantats in den bei der Resektion geschaffenen Raum,
- Aufspreizen des Fusionsimplantats,
- Einbringen eines Führungsdrahts über das Fusionsmodul in ein zu fusionierendes Facettengelenk,
- Aufschieben des transfacettalen Befestigungselements, insbesondere einer kanülierten Schraube, auf den Führungsdraht und Einbringen in das Fusionsmodul,
- Festziehen des transfacettalen Befestigungselements, und
- Festsetzen des Expansionselements.

29. Verfahren nach Anspruch 28, **gekennzeichnet durch** Aufstecken eines kanülierten Schraubendrehers zum Festziehen des transfacettalen Befestigungselements.
